(19) Europäisches Patentamt
European Patent Office
Office européen des brevets

(11) Veröffentlichungsnummer : **0 011 604**
**B1**

(12) # EUROPÄISCHE PATENTSCHRIFT

(45) Veröffentlichungstag der Patentschrift : **22.09.82**

(51) Int. Cl.³ : **C 07 D249/12**, A 01 N 43/64, A 01 N 47/18

(21) Anmeldenummer : **79810146.5**

(22) Anmeldetag : **08.11.79**
**Die Akte enthält technische Angaben, die nach dem Eingang der Anmeldung eingereicht wurden und die nicht in dieser Patentschrift enthalten sind.**

(54) **Triazolylkarbamate, Verfahren zu ihrer Herstellung und ihre Verwendung.**

(30) Priorität : **14.11.78 CH 11690/78**

(43) Veröffentlichungstag der Anmeldung : **28.05.80 (Patentblatt 80/11)**

(45) Bekanntmachung des Hinweises auf die Patenterteilung : **22.09.82 Patentblatt 82/38**

(84) Benannte Vertragsstaaten : **AT BE CH DE FR GB IT NL**

(56) Entgegenhaltungen :
**CH - A5 - 573206**
**DE - A - 2202 471**
**DE - A - 2250 572**
**DE - A - 2412 564**
**DE - A1 - 2457 146**

(73) Patentinhaber : **CIBA-GEIGY AG**
**Patentabteilung Postfach**
**CH-4002 Basel (CH)**

(72) Erfinder : **Gsell, Laurenz, Dr.**
**Maiengasse 56**
**CH-4056 Basel (CH)**
Erfinder : **Ackermann, Peter, Dr.**
**Reichensteinerstrasse 12**
**CH-4153 Reinach (CH)**
Erfinder : **Wehrli, Rudolf, Dr.**
**Dianastrasse 2**
**CH-4310 Rheinfelden (CH)**

**EP 0 011 604 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

## Triazolylkarbamate, Verfahren zu ihrer Herstellung und ihre Verwendung

Die vorliegende Erfindung betrifft Triazolylkarbamatderivate, Verfahren zu ihrer Herstellung und ihre Verwendung in der Schädlingsbekämpfung. Die Triazolylkarbamatderivate haben die Formel

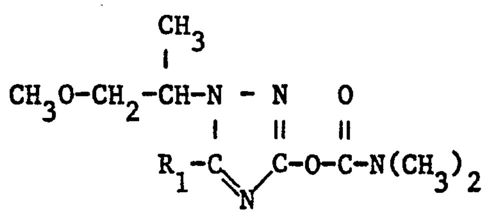

(I)

worin $R_1$ Wasserstoff, Chlor, $C_1$-$C_4$-Alkyl, $C_1$-$C_4$-Alkoxy oder $C_1$-$C_4$-Alkylthio bedeutet.

In der Deutschen Offenlegungsschrift Nr. 2 202 471 sind 1,2,4-Triazolylcarbamate mit insbesondere insektiziden Eigenschaften beschrieben. Die erfindungsgemässen Triazolylkarbamate der Formel I unterscheiden sich von den bekannten Verbindungen durch den Ersatz der unsubstituierten Alkylgruppen in 1-Stellung des 1,2,4-Triazolyls durch den 1-Methyl-2-methoxy-äthylrest und haben demgegenüber eine bessere systemische Wirkung.

Die Alkyl-, Alkoxy- und Alkylthiogruppen bei $R_1$ können geradkettig oder verzweigt sein. Beispiele solcher Gruppen sind u.a.: Methyl, Methoxy, Methylthio, Aethyl, Aethoxy, Aethylthio, n-Propyl, n-Propoxy, n-Propylthio, n-, i-, sek.-, tert.-Butyl, n-Butoxy, i-Propyl, n-Butylthio, iso-Propoxy, iso-Propylthio.

Die Verbindungen der Formel I können nach an sich bekannten Methoden wie folgt hergestellt werden:

A)

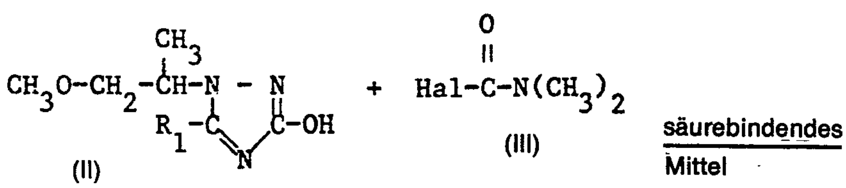


(I)

B)

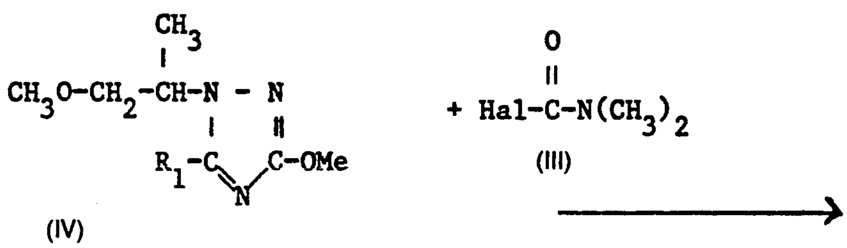


(I)

In den Formeln II bis IV hat $R_1$ die für die Formel I angegebene Bedeutung, Hal steht für Halogen, vorzugsweise für Chlor oder Brom und Me für ein Metall, insbesondere ein Alkalimetall, Ammonium oder Trialkylammonium.

Als säurebindende Mittel kommen beispielsweise folgende Basen in Betracht: tertiäre Amine, wie Triäthylamin, Dimethylanilin, Pyridin, anorganische Basen, wie Hydroxide und Carbonate von Alkali- und Erdalkalimetallen, vorzugsweise Natrium- und Kaliumkarbonat.

Die Verfahren A und B werden bei einer Reaktionstemperatur von 0-120 °C, vorzugsweise bei 20-80 °C, bei normalem Druck und in Lösungs- oder Verdünnungsmitteln durchgeführt. Als Lösungs- oder Verdünnungsmittel eignen sich z.B. Aether und ätherartige Verbindungen, wie Diäthyläther, Dipropyläther, Dioxan, Dimethoxyäthan, Tetrahydrofuran; Amide wie N,N-dialkylierte Carbonsäureamide; aliphatische, aromatische sowie halogenierte Kohlenwasserstoffe, insbesondere Benzol, Toluol, Xylole, Chloroform, Chlorbenzol; Nitrile wie Acetonitril; Dimethylsulfoxyd; Ketone wie Aceton, Methyläthylketon und Wasser.

Die Verbindungen der Formel I eignen sich zur Bekämpfung von verschiedenartigen tierischen und pflanzlichen Schädlingen. So haben sie z.B. nematizide, fungizide und bakterizide Wirkung.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera,

Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von Pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen wie z.B. Musca domestica und Mückenlarven.

Die akarizide bzw. insektizide Wirkung lässt sich durch Zusatz von anderen Insektiziden und oder Akariziden wesentlich verbreitern und an gegebene Umstände anpassen. Als Zusätze eignen sich z.B. org. Phosphorverbindungen ; Nitrophenole und deren Derivate ; Formamidine : Harnstoffe ; pyrethrinartige Verbindungen sowie andere Karbamate und chlorierte Kohlenwasserstoff.

Mit besonderem Vorteil werden Verbindungen der Formel I auch mit Substanzen kombiniert, welche einen hydrotropen Effekt ausüben.

Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfonyl)-propyl)-benzol.

Verbindungen der Formel I können für sich allein oder zusammen mit geeigneten Träger- und/oder Zuschlagstoffen eingesetzt werden. Geeignete Träger- und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Bind- und/oder Düngemittel.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden : Feste Aufarbeitungsformen : Stäubemittel, Streumittel, Granulate (Umhüllungsgranulate, Imprägnierungsgranulate und Homogengranulate) ;

Flüssige Aufarbeitungsformen :

a) in Wasser dispergierbare Wirkstoffkonzentrate :

Spritzpulver (wettable powders), Pasten, Emulsionen ;

b) Lösungen

Der Gehalt an Wirkstoff in den oben beschriebenen Mitteln liegt zwischen 0,1 bis 95 %, dabei ist zu erwähnen, dass bei der Applikation aus dem Flugzeug oder mittels anderer geeigneter Applikationsgeräte Konzentrationen bis zu 99,5 % oder sogar reiner Wirkstoff eingesetzt werden können. Die Wirkstoffe der Formel I können beispielsweise wie folgt formuliert werden (Teile bedeuten Gewichtsteile) :

Stäubemittel : Zur Herstellung eines a) 5 %igen und b) 2 %igen Stäubemittels werden die folgenden Stoffe verwendet :

a) 5 Teile Wirkstoff
95 Teile Talkum ;

b) 2 Teile Wirkstoff
1 Teil hochdisperse Kieselsäure
97 Teile Talkum.

Der Wirkstoff wird mit den Trägerstoffen vermischt und vermahlen.

Granulat : Zur Herstellung eines 5 %igen Granulates werden die folgenden Stoffe verwendet :

5 Teile Wirkstoff
0,25 Teile epoxydiertes Pflanzenöl
0,25 Teile Cetylpolyglykoläther
3,50 Teile Polyäthylenglykol
91 Teile Kaolin (Korngrösse 0,3-0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton in Vakuum verdampft.

Spritzpulver : Zur Herstellung eines a) 40 %igen, b) und c) 25 %igen, d) 10 %igen Spritzpulver werden folgende Bestandteile verwendet :

a) 40 Teile Wirkstoff
5 Teile Ligninsulfonsäure-Natriumsalz
1 Teil Dibutylnaphthalinsulfonsäure-Natriumsalz
54 Teile Kieselsäure

b) 25 Teile Wirkstoff
4,5 Teile Calcium-Ligninsulfonat
1,9 Teile Champagne-Kreide/Hydroxyäthylcellulose-Gemisch (1 : 1)
1,5 Teile Natrium-dibutyl-naphthalinsulfonat
19,5 Teile Kieselsäure
19,5 Teile Champagne-Kreide
28,1 Teile Kaolin ;

c) 25 Teile Wirkstoff
2,5 Teile Isooctylphenoxy-polyäthylen-äthanol
1,7 Teil Champagne-Kreide/Hydroxyäthylcellulose-Gemisch (1 : 1)
8,3 Teile Natriumaluminiumsilikat
16,5 Teile Kieselgur
46 Teile Kaolin ;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10 %igen b) 25 %igen und c) 50 %igen emulgierbaren Konzentrates werden folgende Stoffe verwendet.

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol ;

b) 25 Teile Wirkstoff
2,5 Teile epoxidiertes Pflanzenöl
10 Teile eines Alkylarylsulfonat/Fettalkoholpolyglykol-äther-Gemisches
5 Teile Dimethylformamid
57,5 Teile Xylol ;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol

Aus solchen Konzentraten können durch Verdünnen mit Wasser Emulsionen jeder gewünschten Konzentration hergestellt werden.

Sprühmittel: Zur Herstellung eines a) 5 %igen und b) 95 %igen Sprühmittels werden die folgenden Bestandteile verwendet:

a) 5 Teile Wirkstoff
1 Teil epoxydiertes Pflanzenöl
94 Teile Benzin (Siedegrenzen 160-190 °C) ;

b) 95 Teile Wirkstoff
5 Teile epoxydiertes Pflanzenöl

## Beispiel 1

Herstellung von N,N-Dimethyl-1-(1-methyl-2-methoxy-äthyl)-5-chlor-1,2,4-triazolyl-karbamat

0,1 Mol der Verbindung der Formel

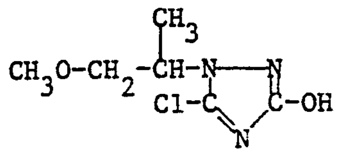

wird mit 0,1 Mol trockenem, pulverisiertem Kaliumkarbonat in 500 ml Methyläthylketon während vier Stunden auf 80 °C erwärmt. Nach dem Erkalten werden bei Raumtemperatur 0,1 Mol Dimethylkarbamoylchlorid zugegeben. Das Reaktionsgemisch wird danach bei 40 °C 12 Stunden gerührt. Nach der Filtration der Salze, dem Entfernen des Lösungsmittels und dem Umkristallisieren des Rohproduktes erhält man die Verbindung der Formel

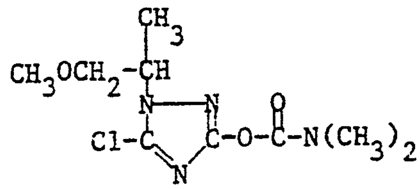

mit einem Schmelzpunkt von 82-84 °C.

Auf analoge Weise werden auch folgende Verbindungen hergestellt :

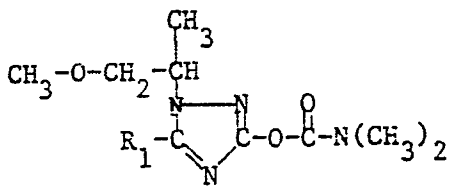

| $R_1$ | |
|---|---|
| $-CH_3$ | Smp. : 105-106 °C |
| H | Smp. : 54- 55 °C |
| $-SCH_3$ | $n_D^{20°}$ = 1,511 0 |
| $-SC_2H_5$ | Smp. : 46-48 °C |
| $-SC_3H_7$(i) | Smp. : 64-67 °C |
| $-OCH_3$ | $n_D^{20°}$ = 1,474 2 |
| $-OC_3H_7$(i) | $n_D^{20°}$ = 1,467 8 |
| $-OC_2H_5$ | $n_D^{20°}$ = 1,470 8 |

## Beispiel 2

### A) Insektizide Frassgift-Wirkung

Baumwollpflanzen wurden mit einer 0,05 %igen wässrigen Wirkstoffemulsion (erhalten aus einem 10 %igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24 °C und 60 % relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

### B) Systemisch-insektizide Wirkung

Zur Feststellung der systemischen Wirkung wurden bewurzelte Bohnenpflanzen (Vicia faba) in eine 0,01 %ige wässrige Wirkstofflösung (erhalten aus einem 10 %igen emulgierbaren Konzentrat) eingestellt. Nach 24 Stunden wurden auf die oberirdischen Pflanzenteile Blattläuse (Aphis fabae) gesetzt. Durch eine spezielle Einrichtung waren die Tiere vor der Kontakt- und Gaswirkung geschützt. Der Versuch wurde bei 24 °C und 70 % relativer Luftfeuchtigkeit durchgeführt.

Verbindungen gemäss Beispiel 1 zeigten im obigen Test systemisch insektizide Wirkung gegen Aphis fabae.

## Beispiel 3

Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, dass kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der « Haltezeit » standen die behandelten Pflanzen in Gewächshauskabinen bei 25°.

Verbindungen gemäss Beispiel 1 wirkten im obigen Test gegen Adulte, Larven und Eier von Tetranychus urticae.

Beispiel 4

Wirkung gegen Bodennematoden

Zur Prüfung der Wirkung gegen Bodennematoden wurden die Wirkstoffe in durch Wurzelzellen-Nematoden (Meloidogyne arenaria) infizierte Erde gegeben und innig vermischt. In die so vorbereitete Erde wurden in einer Versuchsreihe unmittelbar danach Tomatensetzlinge gepflanzt und in einer anderen Versuchsreihe nach 8 Tagen Wartezeit eingesät.

Zur Beurteilung der nematiziden Wirkung wurden 28 Tage nach dem Pflanzen bzw. nach der Saat die an den Wurzeln vorhandenen Gallen ausgezählt. In diesem Test zeigten Wirkstoffe gemäss Beispiel 1 eine gute Wirkung gegen Meloidogyne arenaria.

Beispiel 5

Wirkung gegen Zecken

A) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OPresistenten Larven Versuche durchgeführt. (Die Resistenz bezieht sich auf die Verträglichkeit von Diazinon). Verbindungen gemäss Beispiel 1 wirkten in diesen Tests gegen Adulte und Larven von Rhipicephalus bursa und sensible resp. OP-resistente Larven von Boophilus microplus.

Beispiel 6

Wirkung gegen Erysiphe graminis auf Hordeum vulgare

Ca. 8 cm hohe Gerstenpflanzen wurden mit einer aus Spritzpulver des Wirkstoffes hergestellten Spritzbrühe (0,05 % Aktivsubstanz) besprüht. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen gemäss Beispiel 1 wirkten in diesem Test gegen Erysiphe graminis.

**Ansprüche**

1. Eine Verbindung der Formel

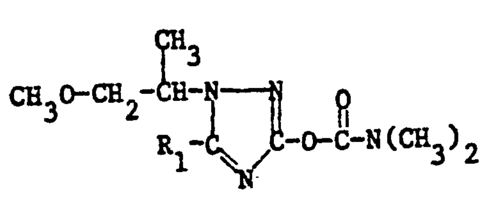

worin $R_1$ Wasserstoff, Chlor, $C_1$-$C_4$-Alkyl, $C_1$-$C_4$-Alkoxy oder $C_1$-$C_4$ Alkylthio bedeutet.

2. Die Verbindung gemäss Anspruch 1 der Formel

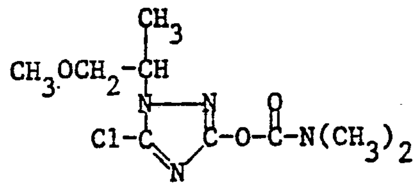

3. Die Verbindung gemäss Anspruch 1 der Formel

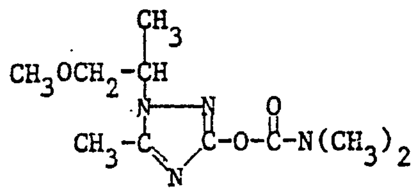

4. Die Verbindung gemäss Anspruch 1 der Formel

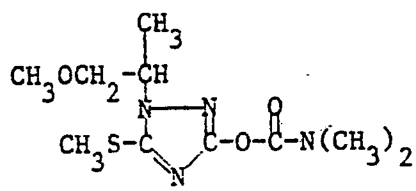

5. Die Verbindung gemäss Anspruch 1 der Formel

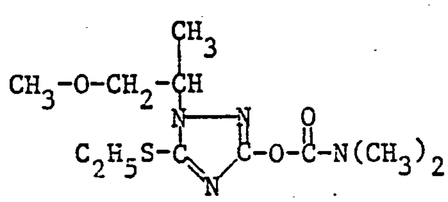

6. Die Verbindungen gemäss Anspruch 1 der Formel

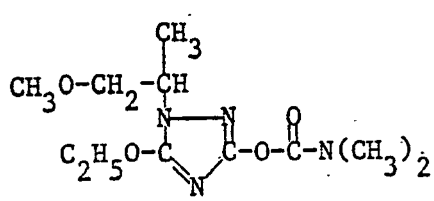

7. Verfahren zur Herstellung von Verbindungen gemäss Anspruch 1, dadurch gekennzeichnet, dass man in an sich bekannter Weise eine Verbindung der Formel

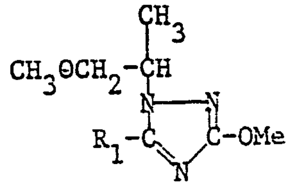

worin $R_1$ die im Anspruch 1 angegebene Bedeutung hat und Me für Wasserstoff oder ein Metallatom steht, gegebenenfalls in Gegenwart eines säurebindenden Mittels mit einer Verbindung der Formel

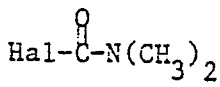

umsetzt, worin Hal für Halogen steht.

8. Ein Schädlingsbekämpfungsmittel, welches neben geeigneten Trägeroder anderen Zuschlagstoffen als aktive Komponente eine Verbindung gemäss Anspruch 1 enthält.

9. Die Verwendung einer Verbindung gemäss Anspruch 1 zur Bekämpfung von verschiedenartigen tierischen und pflanzlichen Schädlingen (ausgenommen die therapeutische Verwendung am menschlichen oder tierischen Körper).

10. Die Verwendung gemäss Anspruch 9 zur Bekämpfung von Insekten und Vertretern der Ordnung Akarina.

**Claims**

1. A compound of the formula

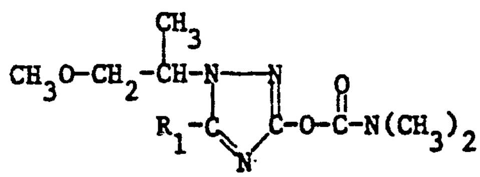

wherein $R_1$ is hydrogen, chlorine, $C_1$-$C_4$-alkyl, $C_1$-$C_4$-alkoxy or $C_1$-$C_4$-alkylthio.

2. The compound according to Claim 1 of the formula

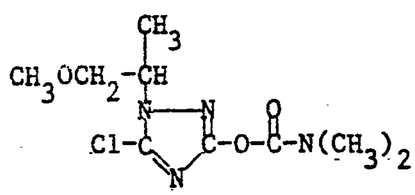

3. The compound according to Claim 1 of the formula

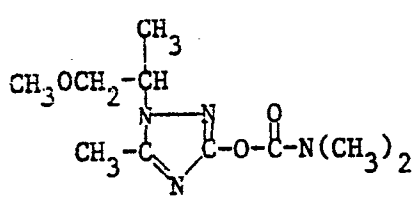

4. The compound according to Claim 1 of the formula

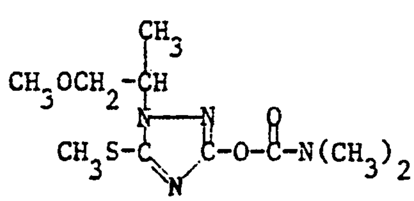

5. The compound according to Claim 1 of the formula

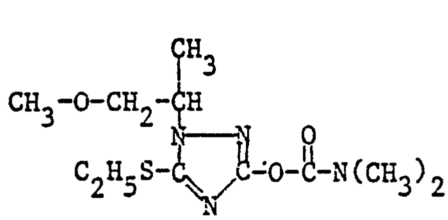

6. The compound according to Claim 1 of the formula

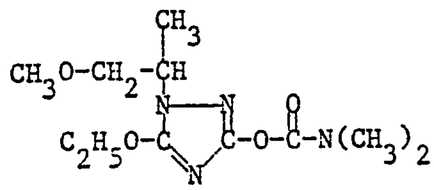

7. A process for producing a compound according to Claim 1, which process comprises reacting, in a manner known per se, a compound of the formula

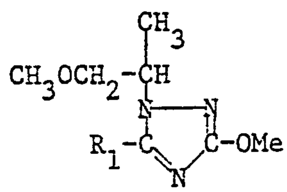

wherein $R_1$ has the meaning defined in Claim 1, and Me is hydrogen or a metal atom, optionally in the presence of an acid-binding agent, with a compound of the formula

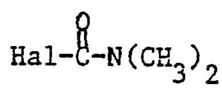

wherein « Hal » denotes halogen.

8. A pesticidal composition which comprises a compound according to Claim 1 as active ingredient, and suitable carriers and/or other additives.

9. The use of a compound according to Claim 1 for combating various animal and plant pests (with the exception of the therapeutic use in the human or animal body).

10. The use according to Claim 9 for combating insects, and representatives of the order Acarina.

**Revendications**

1. Un composé de formule

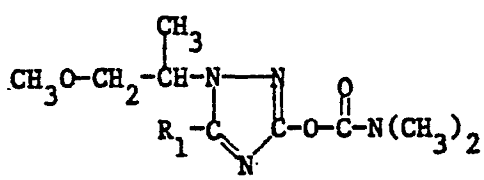

dans laquelle $R_1$ représente l'hydrogène, le chlore, un groupe alkyle en $C_1$-$C_4$, alcoxy en $C_1$-$C_4$ ou alkylthio en $C_1$-$C_4$.

2. Le composé selon la revendication 1, de formule

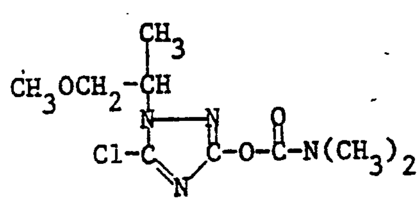

3. Le composé selon la revendication 1, de formule

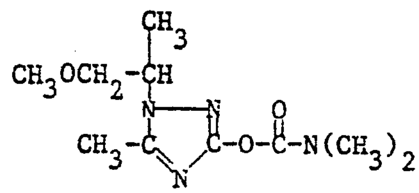

4. Le composé selon la revendication 1, de formule

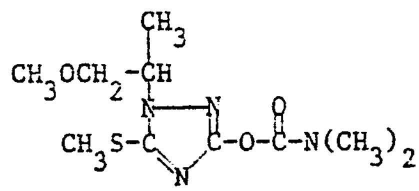

5. Le composé selon la revendication 1, de formule

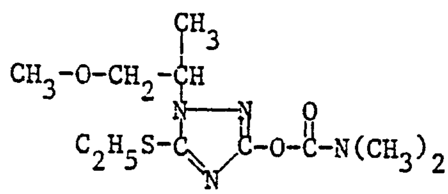

6. Le composé selon la revendication 1, de formule

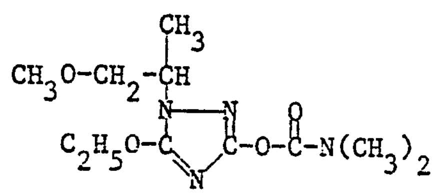

7. Procédé de préparation des composés selon la revendication 1, caractérisé en ce que l'on fait réagir de manière connue en soi un composé de formule

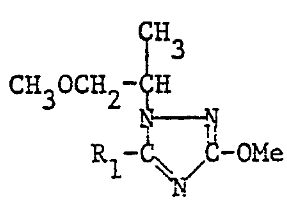

dans laquelle $R_1$ a la signification indiquée dans la revendication 1 et Me représente l'hydrogène ou un atome de métal, le cas échéant en présence d'un agent fixant les acides, avec un composé de formule

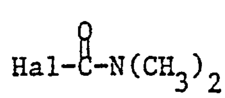

dans laquelle Hal représente un halogène.

8. Un produit pesticide contenant en tant que composant actif, avec des véhicules ou d'autres additifs appropriés, un composé selon la revendication 1.

9. L'utilisation d'un composé selon la revendication 1 pour combattre les parasites animaux et végétaux des types les plus variés (à l'exception de l'utilisation thérapeutique sur l'organisme humain ou animal).

10. L'utilisation selon la revendication 9, pour combattre les insectes et les représentants de l'ordre des acariens.